# EUROPEAN PATENT APPLICATION

(11) **EP 3 789 117 A1**
(43) Date of publication of application: **10.03.2021**
(21) Application number: 19196279.4
(22) Date of filing: 09.09.2019
(51) Int. Cl.: B01L 7/00, B01L 3/00, G01N 21/00

(54) **SMARTPHONE PCR DEVICE**

(71) Applicant: F. Hoffmann-La Roche AG, 4070 Basel (CH); Roche Diagnostics GmbH, 68305 Mannheim (DE)
(72) Inventor: SCHWERTNER, Heiko, 6343 Rotkreuz (CH)
(74) Representative: Merkel, Patrick

(57) **Abstract**

The present disclosure relates to a thermocycling unit for use with a conventional smartphone, as well as methods for the amplification of nucleic acid using the same. The thermocycling unit makes use of several built-in features of the smartphone and thus requires relatively little complexity on the thermocycling unit itself.

## Description

### Field of the Invention

The present invention belongs to the field of analyzing biological samples, such as a sample suspected to contain a pathogen. Within this field, it concerns a device and a method for carrying out a Polymerase Chain Reaction (PCR) in combination with a conventional smartphone.

### Background

In recent years, Point-of-Care (PoC) diagnostics have become of increasing significance and interest. Instead of the often time-consuming and logistically demanding transport of patient samples to central laboratories - or even taking the samples at such sites like, for instance, hospitals - it is in many cases advantageous to obtain diagnostic results in a quicker and more efficient manner. One example would be in-situ diagnosis - from obtaining a patient sample to delivering the diagnostic result - at a doctor's practice using a PoC device that is economically affordable and relative easy to handle. Such an approach also abolishes the risks associated with an elsewise potentially necessary shipping of sample material to a central laboratory, including sample degradation due to stability issues.

However, while existing PoC devices are usually more compact than high- or medium-throughput analyzers or even integrated modular laboratory systems, they are usually still too large to make them available in everyday situations, i.e. outside of a medical site. Moreover, such devices would ideally be battery-powered and thus independent from an external power source.

One of the approaches in the art to solve these problems has involved the use of smartphones, as these devices are almost omnipresent in the modern society. They fulfil the requirements set out above, including portability and independence from an external power supply. For instance, WO 2017/025984 discloses a device for use in conjunction with a smartphone in order to conduct an isothermal, ligase-mediated nucleic acid amplification. While this device exploits certain components of a conventional smartphone, it still exhibits a level of complexity that may contradict a wide use. For example, certain elements of the device disclosed in this reference might render it too expensive to be used as a standard PoC device by patients themselves as opposed to medical personnel at a doctor's practice. The ease of use might also be a limiting factor in practice.

The present disclosure describes devices and methods that may reduce some of the shortcomings in the art.

### Summary

A first aspect of the present disclosure is a thermocycling unit for carrying out a Polymerase Chain Reaction (PCR) in connection with a smartphone having a CPU, a power source, a display, a light source and a camera. The thermocycling unit includes a number of components, such as a housing with an inlet port for a liquid sample, connected to a cyclic internal reaction channel by a gate. Further, the thermocycling unit includes at least two thermal elements thermally connected to the reaction channel and configured to be set to different temperatures, such that they are able to create distinct temperature zones and thus a temperature gradient along the reaction channel. A docking mechanism is present in order to dock the thermocycling device to the smartphone, while an optical path is configured to guide light from the smartphone's built-in light source through a window via the reaction channel to the camera of the smartphone to facilitate detection of the amplified nucleic acid.

Another aspect disclosed herein is a method for conducting a PCR on a liquid biological sample. The method commences with injection of the liquid sample into the thermocycling unit described herein via its liquid sample inlet port and docking the thermocycling unit to a conventional smartphone with a CPU, a power source, a display, a light source and a camera. By setting the thermal elements to different temperatures, a temperature gradient is created between the resulting distinct temperature zones, facilitating the cycling of the liquid sample through the reaction channel by thermal convection. While the sample passes the different temperature zones, a PCR is conducted as the distinct temperatures trigger the steps of a conventional PCR cycle. During and/or after the PCR, a light beam from the smartphone's light source is directed through the reaction channel and into the camera of the smartphone, thereby facilitating detection of the amplified nucleic acid.

Other aspects described herein are the use of the thermocycling unit described herein for conducting a PCR, and an analytical system including the thermocycling unit described herein and a smartphone having a CPU, a power source, a display, a light source and a camera.

### Short description of the Figures

Fig. 1A shows a perspective view of an embodiment of the fluidic channel system of the thermocycling unit disclosed herein, along with its heating elements.
**Fig. 1B** displays the fluidic system of Fig. 1A embedded in its housing (10), the latter depicted in a transparent manner for the sake of clarity.
**Fig. 1C** provides a perspective view of the assembled housing (10) from the outside.
**Figs. ID** and **IE** are perspective depictions of the lower (13) and upper (14) portions of the housing (10).
**Figs. 2A-D** represent schematic drawings of exemplary embodiments of the optical paths for detection of nucleic acids amplified within the thermocycling unit (1) disclosed herein.
**Figs. 3A-E** show embodiments of the fluidic system of the thermocycling unit (1) described herein in a schematic depiction viewed from above.

### Detailed description

A first aspect of the present disclosure is a thermocycling unit for carrying out a Polymerase Chain Reaction (PCR) in connection with a smartphone having a CPU, a power source, a display, a light source and a camera, the thermocycling unit comprising:
- a reaction channel providing a cyclic fluidic path connected to a liquid sample inlet port via a gate;
- a housing comprising the liquid sample inlet port and an optical interface configured to be placed onto the light source and the camera of the smartphone, the optical interface being connected to an optical path within the thermocycling unit for guiding light from the light source via the reaction channel to the camera of the smartphone;
- a data transfer port and an energy transfer port, wherein the data transfer port and the energy transfer port may be combined or separate;
- an electric circuitry comprising a first and a second thermal element in thermal contact with the reaction channel, wherein the first thermal element is spatially separated from the second thermal element, and wherein the first and the second thermal elements are configured to be set to different pre-determined temperature values such as to establish distinct temperature zones and a temperature gradient along the reaction channel;
- a docking mechanism for reversibly docking the thermocycling unit to the smartphone.

A "smartphone", in the context of the present disclosure, means a handheld personal computer with a mobile operating system and an integrated mobile broadband cellular network connection for voice, SMS, and internet data communication. Most, if not all, smartphones also support Wi-Fi. Smartphones are typically pocket-sized, as opposed to tablet computers, which are much larger. Modern smartphones usually have a touchscreen color display with a graphical user interface that covers the front surface and enables the user to use a virtual keyboard to type and press onscreen icons to activate and/or manage apps (software components).

Typical functionalities of a smartphone include a telephone, digital camera and video camera, GPS navigation, a media player, clock, news, calculator, web browser, handheld video game player, flashlight, compass, an address book, note-taking, digital messaging, an event calendar, etc. Typical smartphones include one or more of the following sensors: magnetometer, proximity sensor, barometer, gyroscope, or accelerometer. Since 2010, smartphones have adopted integrated virtual assistants, such as Apple Siri, Amazon Alexa, Google Assistant, Microsoft Cortana, BlackBerry Assistant and Samsung Bixby. Most smartphones produced from 2012 onward have high-speed mobile broadband 4G LTE.

The smartphone used in conjunction with the thermocycling unit disclosed herein comprises a CPU, a power source, a display, a light source and a camera. These components are used for conducting the PCR within the thermocycling unit.

A central processing unit (CPU), as used herein, is the electronic circuitry within a computer - which is in the present disclosure a smartphone - that carries out the instructions of a computer program (or app) by performing the basic arithmetic, logical, control and input/output (I/O) operations specified by the instructions. Traditionally, the term "CPU" refers to a processor, more specifically to its processing unit and control unit (CU), distinguishing these core elements of a computer from external components such as main memory and I/O circuitry.

The "power source" supplies the smartphone - as well as certain components of the thermocycling unit disclosed herein - with energy. In most cases, the power source of the smartphone in the context described herein is a rechargeable electric battery, in some embodiments a lithium-ion battery. An "electric battery" is a device consisting of one or more electrochemical cells with external connections provided to power electrical devices such as flashlights, smartphones, and electric cars. As opposed to single-use (primary) batteries, so-called secondary batteries are rechargeable and therefore typically the batteries of choice for devices like a smartphone. Such batteries can be discharged and recharged multiple times using an applied electric current; the original composition of the electrodes can be restored by reverse current. Examples include the lead-acid batteries used in vehicles and the above-mentioned lithium-ion batteries used for portable electronics such as laptops and smartphones.

In some embodiments of the thermocycling unit disclosed herein, the first (115) and/or the second thermal element (116) are controlled via the CPU and powered by the power source of the smartphone (2).

As used herein, the term "Polymerase Chain Reaction" (PCR) refers to a method for amplification well known in the art for increasing the concentration of a segment of a target polynucleotide in a sample, where the sample can be a single polynucleotide species, or multiple polynucleotides. Generally, the PCR process consists of introducing a molar excess of two or more extendable oligonucleotide primers to a reaction mixture comprising the desired target sequence(s), where the primers are complementary to opposite strands of the double stranded target sequence. The reaction mixture is subjected to a program of thermal cycling in the presence of a DNA polymerase, resulting in the amplification of the desired target sequence flanked by the DNA primers. Such thermal cycling can be facilitated by the thermocycling unit as described herein in conjunction with a Reverse transcriptase PCR (RT-PCR) is a PCR reaction that uses RNA template and a reverse transcriptase, or an enzyme having reverse transcriptase activity, to first generate a single stranded DNA molecule prior to the multiple cycles of DNA-dependent DNA polymerase primer elongation. Multiplex PCR refers to PCR reactions that produce more than one amplified product in a single reaction, typically by the inclusion of more than two primers in a single reaction. Generally, a PCR cycle comprises a denaturation step at usually above 90 °C in which the individual strands of a double-stranded template nucleic acid are separated from each other. At a significantly lower temperature, often between 50 °C and 65 °C, the primers anneal to their complementary target sites on the respective templates strands, before the temperature is ramped up again to mostly between 70 °C to 80 °C, where the thermostable nucleic acid polymerase exhibits its maximum enzyme activity for extending the nascent amplicon. Methods for a great variety of PCR applications are widely known in the art, and described in many sources, for example, Ausubel et al. (eds.), Current Protocols in Molecular Biology, Section 15, John Wiley & Sons, Inc., New York (1994).

The devices and methods described herein are not strictly limited to conservative PCRs. With certain modifications, the skilled person will also be able to carry out different methods for the amplification of nucleic acids. Such amplification reactions comprise, among others, the Ligase Chain Reaction, Polymerase Ligase Chain Reaction, Gap-LCR, Repair Chain Reaction, 3SR, NASBA, Strand Displacement Amplification (SDA), Transcription Mediated Amplification (TMA), and Qβ-amplification.

It is further conceivable that the device disclosed herein may be useful in the context of antibody-based assays. In this case, the device might be embodied as a less complex system not requiring cycling of a liquid sample through multiple different dedicated temperature zones.

Also, the thermocycling unit described herein is useful for both qualitative and quantitative nucleic acid amplification reactions.

Qualitative detection of a nucleic acid in a biological sample is, for instance, crucial for recognizing an infection of an individual. Thereby, one important requirement for an assay for detection of a microbial infection is that false-negative or false-positive results be avoided, since such results would almost inevitably lead to severe consequences with regard to treatment of the respective patient. Thus, especially in PCR-based methods, a qualitative internal control nucleic acid is often added to the detection mix. Said control is particularly important for confirming the validity of a test result: At least in the case of a negative result with regard to the respective target nucleic acid, the qualitative internal control reaction has to perform reactive within given settings, i.e. the qualitative internal control must be detected, otherwise the test itself is considered to be inoperative. However, in a qualitative setup, said qualitative internal control does not necessarily have to be detected in case of a positive result. For qualitative tests, it is especially important that the sensitivity of the reaction is guaranteed and therefore strictly controlled. As a consequence, the concentration of the qualitative internal control must be relatively low so that even in a situation of slight inhibition the qualitative internal control is not detected and therefore the test is invalidated.

On the other hand, and in addition to mere detection of the presence or absence of a target nucleic acid in a sample, it is often important to determine the quantity of said nucleic acid. As an example, stage and severity of a viral disease may be assessed on the basis of the viral load. Further, monitoring of any therapy requires information on the quantity of a pathogen present in an individual in order to evaluate the therapy's success. For a quantitative assay, it is necessary to introduce a quantitative standard nucleic acid serving as a reference for determining the absolute quantity of a target nucleic acid. Quantitation can be effectuated either by referencing to an external calibration or by implementing an internal quantitative standard.

The thermocycling unit disclosed herein allows for classical endpoint PCR (affording qualitative and semi-quantitative detection) as well as realtime PCR (both qualitative and quantitative assays). The setup described herein enables the skilled person, among other things, to monitor the reaction taking place in the reaction channel continuously or in defined intervals. For instance, in a PCR using fluorescence-labeled 5'-nuclease probes (TaqMan format), the increasing concentration of the respective amplicon may be monitored via the optical path of the thermocycling unit described herein, thereby enabling analysis of a PCR growth curve, as known by the person skilled in the art.

Therefore, an aspect of the present disclosure is the use of the thermocycling unit described herein for qualitative and/or quantitative purposes. In some embodiments, the thermocycling unit described herein is used in quantitative PCR, in some embodiments quantitative realtime PCR.

A "thermal element", as used herein, is an object that can actively or passively adjust its temperature and, in consequence, the temperature of its surroundings. In some embodiments, the thermal elements comprise or consist of Peltier elements. Also in some embodiments, they comprise or consist of electrical resistance elements such as resistors. In further embodiments, they comprise or consist of electrodeposited thermoelectric elements. An "electrodeposited thermoelectric element" relates to a thermoelectric element made or manufactured by electrodeposition of the p-type and n-type elements. Electrodeposition is a processes which includes electrocoating, e-coating, cathodic electrodeposition, anodic electrodeposition, and electrophoretic coating, or electrophoretic painting. A characteristic feature of this process is that colloidal particles suspended in a liquid medium migrate under the influence of an electric field (electrophoresis) and are deposited onto an electrode. All colloidal particles that can be used to form stable suspensions and that can carry a charge can be used in electrophoretic deposition. This includes materials such as polymers, pigments, dyes, ceramics, silicates, metalloids (= semimetals) and metals. The process is useful for applying materials to any electrically conductive surface. As electrodeposition enables a highly granular arrangement of the p-type and n-type elements electrodeposited thermoelectric element have a high flexibility and may be individually shaped with rather low effort, which is particularly advantageous in view of the limited amount of space available in connection with a mobile device such as a smartphone. Relatively small electrodeposited thermoelectric elements can be applied to the inside of the thermocycling unit disclosed herein, and they possess mechanical flexibility which may be required within the housing thereof.

The "first thermal element" and the "second thermal element" are distinct elements spaced apart from each other such that they can establish a temperature gradient between one another when connected to a thermally conducting medium, such as the cyclic fluidic path of the thermocycling unit described herein. Since a conventional PCR, as described above, usually relies on three different temperatures for denaturing, annealing, and extending, some embodiments of the thermocycling unit described herein include a third thermal element. In some of these embodiments, the first thermal element is configured to be heated to a denaturing temperature (about 90 to 95 °C), the second thermal element is configured to be heated to an annealing temperature (about 50 to 65 °C), and the third thermal element is configured to be heated to an extending temperature (about 70 to 80 °C).

The "docking mechanism" for reversibly docking the thermocycling unit to the smartphone facilitates the physical connection between these two components. "Reversibly docking", as used herein, means that docking between the respective structures can be unmade without destruction of any of the components. For instance, the thermocycling unit may comprise a flexible skirting at or along its rim which may be engaged into a press-fit on the frame of the smartphone. In some embodiments, the docking mechanism comprises one or more elements selected from the group consisting of a snap fit, a press fit, latches, and hooks. Other suitable structures are conceivable by the person skilled in the art.

The thermocycling unit described herein combines a number of advantages as compared to other PoC devices described in the art.

For instance, it exploits a high percentage of the standard components of a conventional smartphone, thus abolishing the need for many components that would else have to be provided by the thermocycling unit. As a consequence, the thermocycling unit can be produced at lower cost and exhibit a lower overall complexity. In fact, the thermocycling unit disclosed herein may even be produced as a disposable item. Single use of a thermocycling unit described herein may be beneficial for the avoidance of the risk of cross-contamination, which is often one of the challenges of the typically very sensitive Polymerase Chain Reaction. An individual may carry multiple disposable thermocycling units with him- or herself, for instance, when traveling through remote regions and/or being disposed to certain pathogens.

In the context of the invention, "disposable" or "consumable" means a component which is used only a limited number of times, in some embodiments only once, and is then discarded. In the context of the present disclosure, the thermocycling unit described herein is in some embodiments disposable. Hence, it can be used as described herein, and then it may be discarded after conducting a defined number of PCRs, for example, one PCR, or multiple PCRs. As discussed above, cross-contamination is an important factor in connection with PCR, which is why the single use of the thermocycling unit described herein may be particularly advantageous. A thermocycling unit having been exposed to a biological sample, such as a blood sample, is discarded and does therefore not need to be cleaned, i.e., decontaminated.

The thermocycling unit disclosed herein may be made of a number of suitable materials. For instance, it may be made of a metal or an alloy, or a non-metallic material, or a combination thereof. Among non-metals, plastics or hard plastics are suitable materials, such as a cyclic olefin polymer (COP) or copolymer (COC). Other suitable materials include polypropylene (PP), polyoxymethylene (POM), polycarbonate (PC), acrylonitrile butadiene styrene (ABS), or even non-plastic materials.

A thermocycling unit made of metal may display the advantage of high robustness. Such a design may be especially advantageous in embodiments where the thermocycling unit described herein is used as a durable component as opposed to a disposable item. In the case of a disposable component, on the other hand, other materials may be of advantage, such as the plastics mentioned above.

While, when using the thermocycling unit disclosed herein, a quick analysis may be performed, for instance, whether certain pathogenic nucleic acids are detectable in an individual, the respective result would not necessarily need to represent the final diagnosis. In such a case, the individual may be prompted by a positive result - detection of the pathogen in question - to subject him- or herself to testing at a medical facility. However, in case of a negative result, this may not be necessary.

Alternatively, the experimental data - whether raw or processed - may be forwarded to a healthcare professional. Such transfer may advantageously make use of the smartphone as a communication device. For instance, data may be transferred via a secure wireless connection to a medical facility. In some cases, these data may be interpreted by the corresponding healthcare professional, and the diagnostic result may be sent back to the individual.

Also, in some embodiments, the reaction channel of the thermocycling unit described herein may be pre-filled with PCR reagents.

Such reagents are known to the person skilled in the art and comprise specific and unspecific primers and probes, deoxynucleotides such as dNTPs, buffers, bivalent cations such as Mg²⁺ or Mn²⁺, DNA polymerases with or without exonuclease activity, reverse transcriptases, dyes such as fluorescent dyes or quenchers which may or may not be attached to a primer or a probe, aptamers, or other components known to the skilled person. In some embodiments, these reagents may comprise primers and probes specific for a certain target nucleic acid. For instance, a thermocycling unit may be pre-filled with primers and probes specifically hybridizing to a nucleic acid sequence of Methicillin-Resistant Staphylococcus Aureus (MRSA), while another thermocycling unit may be pre-filled with primers and probes specifically hybridizing to a nucleic acid sequence of Human Immunodeficiency Virus (HIV). An individual may thus carry a certain set of thermocycling units dedicated to detecting specific distinct target nucleic acids, such as pathogenic nucleic acids, depending, for example, on which pathogens the individual may be exposed to in a certain situation. It is particularly advantageous to design such pre-filled thermocycling units as disposable components. For instance, members of a humanitarian or medical organization could be equipped with sets of disposable thermocycling units as disclosed herein, matching the range of pathogens present in the area of their respective operation.

However, the thermocycling unit disclosed herein is not restricted to raw samples such as untreated whole blood of an individual. A primary sample may also be pre-treated in order to isolate nucleic acids, upon which a solution containing the latter may be introduced into the thermocycling unit disclosed herein and processed as described herein, in some embodiments, without the need for any adaptions of the method described herein.

In the context of the present disclosure, the terms "isolation", "purification" or "extraction" of a biological target material relate to the following: Before biological target materials like, for instance, nucleic acids may be analyzed in a diagnostic assay by amplification or the like, they typically have to be purified, isolated or extracted from biological samples containing complex mixtures of different components. In the context described herein, the mycobacteria, if present in the sputum sample, contain a variety of different biomolecules, and in many cases only a subgroup of these molecules are of interest for a given type of analysis. For instance, nucleic acids to be analyzed by PCR in a downstream process may need to be separated from the other biomolecules present in the mixture. Suitable methods for isolation are known to the person skilled in the art.

Typically, one of the first steps comprises releasing the contents of cells or viral particles, for example, by using enzymes and/or chemical reagents. This process is commonly referred to as lysis. For enrichment of the analyte in question in the lysate, one useful procedure for binding nucleic acids entails the selective binding of nucleic acids to glass or silica surfaces of binding particles such as magnetic particles in chaotropic salt solutions and separating the nucleic acids from contaminants such as agarose, proteins or cell debris. The composition used herein may be especially useful when nucleic acids are bound to glass surfaces, as the composition already contains a chaotropic agent which is usually necessary for such binding.

Also an aspect of the present disclosure is a method for conducting a PCR on a liquid biological sample, the method comprising the steps of:
a) introducing the liquid biological sample into the liquid sample inlet port of the thermocycling unit described herein and docking the latter to a smartphone having a CPU, a power source, a display, a light source and a camera;
b) setting the temperatures of the first and the second thermal element of the thermocycling unit, by using the CPU and the power source of the smartphone, to different values and thereby establishing distinct temperature zones and a temperature gradient along the reaction channel;
c) cycling the liquid biological sample through the distinct temperature zones of the reaction channel by using the temperature gradient for thermal convection, thereby conducting the PCR steps of denaturing, annealing and elongating within the distinct temperature zones;
d) detecting a nucleic acid amplification product of the PCR by guiding light emitted from the light source of the smartphone through the optical path of the thermocycling unit to the camera of the smartphone either during or after step c).

The method described above enables the skilled person to exploit the advantageous structures of the thermocycling unit in order to conduct a PCR using a conventional smartphone. In some embodiments, the method described above does not require any additional steps.

Also in some embodiments, the method further includes processing of the data generated during or after the PCR. In such embodiments, the data may be conveniently presented to the user by displaying it on the smartphone's screen.

Therefore, in some embodiments, the method disclosed herein further comprises after step d) the step of processing the detection data and outputting a result to the display of the smartphone.

Regarding the injection of the sample from the sample inlet port through the gate until reaching the reaction channel, some embodiments involve, as mentioned herein, processing of the sample.

Hence, in some embodiments, the method disclosed herein further comprises the step of pre-processing the liquid biological sample after insertion into the thermocycling unit through the liquid sample inlet port, but before cycling the liquid biological sample through the reaction channel.

In a more specific embodiment, the pre-processing step comprises the separation of blood plasma from a whole blood sample.

Advantages of such embodiments have been described herein in the context of the thermocycling unit.

When functionally connected to each other, the smartphone and the thermocycling unit disclosed herein work together as an integrated functional unit.

Therefore, an aspect of the present disclosure is analytical system for conducting a PCR, the system comprising:
- a thermocycling unit as disclosed herein;
- a smartphone having a CPU, a power source, a display, a light source and a camera.

The described embodiments of the thermocycling unit, the analytical system, the method and the use disclosed herein are applicable to any of the above-listed categories. For the avoidance of doubt, for example, an embodiment described in the context of the thermocycling unit described herein is also applicable to the analytical system or the method disclosed herein, and so forth.

### Exemplary embodiments

The following exemplary embodiments are meant to illustrate specific aspects and variants of the thermocycling unit (1) disclosed herein, while they are not limiting.

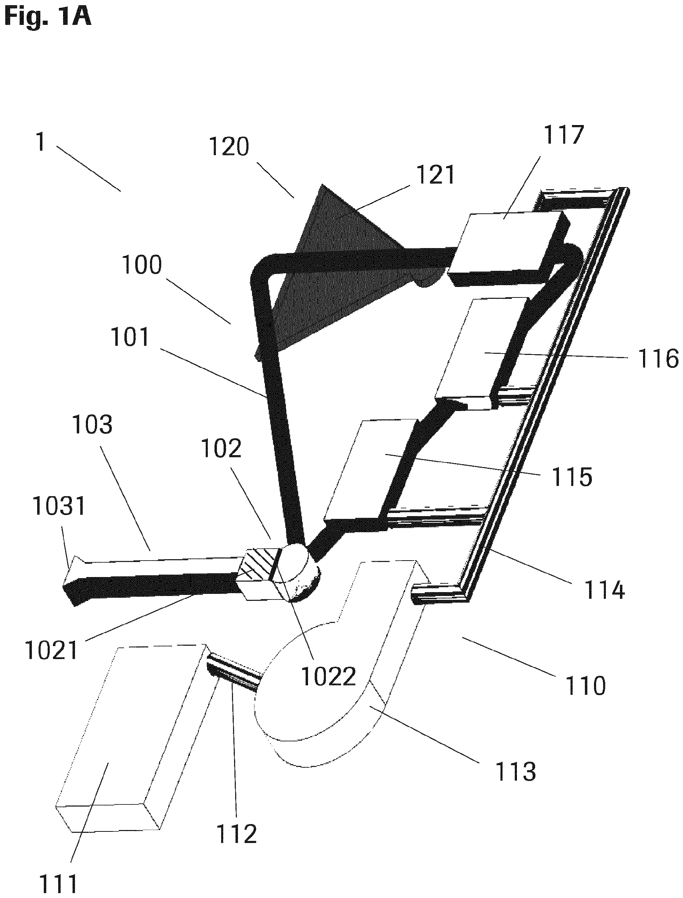

**Fig. 1A** shows a perspective view of the interior of the thermocycling unit (1) described herein. While working as a functional unit, the interior may be roughly divided into a reaction channel (100) for liquid assay components, electric circuitry (110) for thermal control and data management, and an optical path (120) for detection.

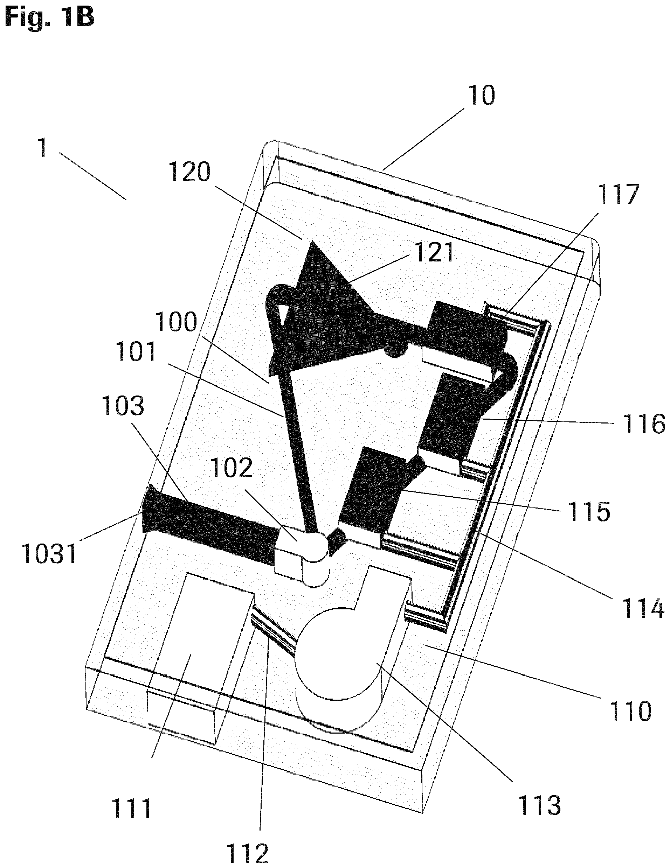

**Fig. 1B** shows the same elements within a housing (10), in this depiction transparent for the sake of clarity.

The reaction channel (100) represents the fluidic system in which the biochemical reaction takes place. The liquid sample to be analyzed is contacted and incubated with the reagents for thermal amplification of nucleic acids within the cyclic fluidic path (101) of the reaction channel (100). In the depicted embodiment, the cyclic fluidic path (101) has a triangular shape. The skilled person will appreciate that other geometrical shapes are also conceivable, such as circular or polygonal shapes. In some embodiments, the reaction channel (100) is made of glass or transparent plastics. In further embodiments, the reaction channel (100) is a glass capillary or an arrangement of glass capillaries. The cyclic fluidic path (101) may, in some embodiments, have a total length of between 0.5 cm to 15 cm, or between 1 cm and 10 cm, or between 2.5 cm and 7.5 cm, or about 5 cm. The outer diameter of the cyclic fluidic path (101) may, in some embodiments, be between 0.5 mm and 5 mm, or between 1 mm and 3.5 mm, or about 2 mm. The volume of the cyclic fluidic path may, in some embodiments, be between 25 µl and 500 µl, or between 50 µl and 350 µl, or about 200 µl. The cyclic fluidic path (101) is fluidically connected to the liquid sample inlet port (103) via a gate (102), in this depiction embodied as a separation unit. At its distal end (in relation to the cyclic fluidic path (101)), the sample inlet port (103) widens into an opening (1031) for the liquid sample to be introduced. The opening (1031) has, in some embodiments, dimensions such as to enable the introduction of a lancet, a glass capillary or similar devices for withdrawing and transferring blood from an individual. The widening shape of the opening (1031) seen in the depicted embodiment contributes to guiding the lancet into the inlet port (103) and thereby reducing the risk of sliding off and/or even breaking it while potentially spilling the blood sample. The proximal end of the inlet port (103), again in relation to the cyclic fluidic path (101), leads into the separation unit (102) which may comprise an absorptive element (1021), such as sponge-like material or fleece. Towards the inlet port (103) or its opening (1031), respectively, the absorptive element (1021) may be sealed by an adhesive strip or another type of barrier which may be removed immediately before introduction of the liquid sample. For instance, an adhesive strip may include a flap protruding from the housing (10) through a slit for convenient reach from the outside. In such an embodiment, the absorptive element (1021) inside the separation unit (102) may be in fluid contact with the inside of the cyclic fluidic path (101), such that the absorptive element (1021) may already be soaked with the liquid biochemical reagents inside the fluidic path (101) in embodiments where the latter is pre-filled with the respective reagents for a given PCR assay. In other embodiments, a second barrier may be included between the separation unit (102) and the cyclic fluidic path (101), such the liquid sample may first be drawn into the absorptive element (1021) of the separation unit (102) by capillary forces upon removal of the first seal, then come into contact with the PCR reagents in the fluidic path (101) upon removal of the second seal. In an alternative embodiment, the seal or the seals may be embodied as frangible membranes which may readily be pierced by a blood withdrawal device such as a lancet. In such an embodiment, an absorptive element (1021) may not be required. The reagent mixture is shielded inside the cyclic fluidic path (101) by the membrane and thereby protected from drying out.

In other embodiments, the biochemical reagents may be pre-disposed inside the fluidic path (101) in dried form, such as freeze-dried or vacuum dried. This is an established method in the art, increasing stability and shelf-life of the reagents. The inlet port (103) or the separation unit (102), respectively, may still comprise a membrane. Whenever the membrane is fractured by introduction of, for instance, a lancet, the liquid sample being introduced will dissolve the predried reagents such that the biochemical reactions required for a PCR experiment may occur. Upon fracturing the membrane, for instance, the liquid sample may be accelerated into the reaction channel (100). A backflow preventer (104) may contribute to maintaining the liquid sample or reaction mixture within the reaction channel (100) or its cyclic fluidic path (101), respectively.

In further embodiments, either instead of or in addition to the components of the separation unit (102) described above, the latter may comprise a blood plasma separation filter (1022). For whole blood samples, it can be advantageous, as described above, to separate the cellular/particular components from the liquid matrix. In other words, blood cells are held back by a blood plasma separation filter (1022) such that blood plasma is obtained. In some embodiments of the thermocycling unit (1) disclosed herein, a whole blood sample may be introduced into the opening (1031) of the inlet port (103) and be subjected to filtering through a plasma separation filter (1022) within the separation unit (102) of the reaction channel (100). Hence, substantially or at least partially purified blood plasma may reach the cyclic fluidic path (101) through the separation unit (102). The advantages of using blood plasma for many (bio)chemical reactions - including PCR - are well known in the art. Whole blood, with its corpuscular components, bears a risk of interfering with such reactions especially on a microscale level. For instance, blood clotting may occur within the reaction channel (100) and obstruct the passage of sample and reagents.

Upon entering the cyclic fluidic path (101), the liquid sample, in the current embodiment now at least partially purified blood plasma, flows in the direction of the temperature gradient established by the heating elements (115, 116, 117). The depicted embodiment includes, in addition to a first (115) and a second (116), also a third thermal element (117). As described above, a conventional PCR typically includes incubation of the respective reaction mixture at three distinct temperatures, such that each of the thermal elements 115 to 117 corresponds to a denaturation (115), an annealing (116), and an elongation zone (117), respectively. The reaction mixture passing first through the denaturation zone (115) represented by the first thermal element is incubated at a temperature sufficiently high to separate double stranded nucleic acids, if present in the liquid sample, into single strands. Following the temperature gradient, the reaction mixture then reaches the second thermal element (116) which is configured to provide a temperature suited for annealing of the PCR primers and, in some embodiments, the PCR probes, to the single-stranded nucleic acid templates. Since an annealing temperature is lower than a denaturation temperature, the method described herein may benefit from the circumstance that the distinct thermal elements are spaced apart from each other. For instance, in the case of transfer from the first (115) to the second thermal element (116), the portion of the reaction mixture situated between these elements is mostly exposed to ambient temperature, thermal conduction facilitated by the typically thin walls of the capillaries (usually made from glass or transparent plastics). In some embodiments the capillary walls have a thickness of between 10 µm and 1 mm, or between 50 µm and 0.5 mm, or about 100 µm. In consequence, this portion of the reaction mixture may cool down from the denaturation temperature to some extent before reaching the second thermal element (116) with its pre-set annealing temperature, thus contributing to the system's efficiency. For instance, a portion of the reaction mixture heated to about 94° C by the first thermal element (115) may be already cooled down to a temperature close to the annealing temperature, for instance, at about 60° C, on its way to the second thermal element (116). In addition to the cooling effects of the ambient temperature, some embodiments comprise further thermal elements between the ones described above that represent the actual PCR cycling zones. For example, a fourth thermal element (not shown) may be incorporated between the first (115) and the second thermal element (116) such as to actively pre-cool the respective portion of reaction mixture. For this purpose, such a fourth thermal element would typically be set to a temperature below the annealing temperature of the second thermal element (116) in order to effect a quicker cooling down of the reaction mixture. The fourth thermal element may, for example, be set to a temperature of about 45° C, or lower.

Similarly, when moving from the annealing zone represented by the second thermal element (116) towards the third thermal element (117) designated for elongation, yet another thermal element (not shown) might be implemented between the second (116) and the third (117) thermal element. This fifth thermal element might, for instance, maintain a temperature between the annealing and the elongation temperature such that the temperature of the respective portion of reaction mixture is actively kept from cooling down towards ambient temperature.

In each PCR cycle after having passed the elongation zone represented by the third thermal element (117), the reaction mixture reaches an optical path (120) for detection, in the depicted embodiment including a prism (121) as an optical element. In further embodiments, more than one prism and/or other optical elements such as lenses or mirrors may be comprised by the optical path (120). Some embodiments of the latter are described elsewhere herein in more detail.

In some embodiments of the thermocycling unit described herein, the optical path (120) comprises one or more elements selected from the group consisting of a prism, a lens, a guiding rod, an optical filter, a diffraction grating, and a mirror.

In brief, the light source of the smartphone is optically coupled to the optical path (120). For instance, in the case of fluorescence-based detection of the amplicon, the light from the smartphone is guided to the portion of the reaction mixture passing the optical path (120). Via an optical filter or the like, the reaction mixture may be exposed to light of a desired excitation wavelength. The light emitted in response by a suitable fluorophore of the nucleic acid to be analyzed or its detection probe can then be passed through another filter permitting the passage of the emission wavelength, and ultimately reach the smartphone camera where the generated signal can be detected. A suitable computer program (app) installed on the respective smartphone may then process and/or analyze the detected signal, or raw data may be transmitted for analysis via a mobile internet connection to a healthcare professional or a central laboratory, or the like.

The reaction channel (100) or its cyclic fluidic path (101), in the depicted embodiment a triangular capillary system, may be embodied as a removable and, in some embodiments, disposable unit. In such embodiments, the thermocycling unit (1) disclosed herein provides a reusable scaffold including the required electric circuitry (110) and the optical path (120). The sample inlet port (103) and/or the gate (102) may be provided already in physical connection with the removable cyclic fluidic path (101) thus forming a complete removable reaction channel (100), or as a separate unit, or else as a reusable part integral to the above-described scaffold thermocycling unit (1).

Embodiments with a removable reaction channel (100) display several advantages. For instance, cyclic fluidic paths (101) such as capillary circles or triangles or other polygonal structures may be provided pre-filled with reagents necessary for a PCR, in some embodiments including reagents for specific assays such as target-specific oligonucleotides. The scaffold might then be produced, for instance, in a costlier manner and thus with higher quality since it would be reused. Moreover, a user would not have to carry multiple thermocycling units (1) for potential multiple uses and different assays, but one scaffold unit would be sufficient while a multiplicity of pre-filled cyclic fluidic paths (101) could be carried, the latter taking up less space.

In other embodiments, the thermal cycling unit (1) is arranged and provided as a complete device, in some embodiments ready-to-use, including the reaction channel (100) as an integral unit. Advantages conferred by such embodiments include the abolishment of the necessity for the user to exchange a capillary system containing a biological sample, including a potential risk of unintended breakage.

Figures 1A and 1B also show the electric circuitry (110) of the depicted embodiment, including the thermal elements (115, 116, 117) described above. The latter are electrically supplied via a cable conduit (114) having cables running through cable chamber 113, another conduit (112) to the energy transfer port (111a), in this embodiment included by a USB port (111). The latter is a convenient solution as it enables the combination of energy transfer and data transfer through the same port, as is the case in many other electrical devices. Most significantly for the devices and methods described herein, most if not all contemporary smartphones have a standard USB port for both energy and data transfer.

Therefore, in some embodiments of the thermocycling unit (1) disclosed herein, the data transfer port (111a) and the energy transfer port (111b) are combined in a USB port (111). At least, as is the case for some smartphones, other ports serving the same purpose (for instance, microUSB or Lightning ports), may be readily connected to a standard USB port via commercially available adaptors. Hence, the functions of the thermocycling unit (1) described herein may be powered by the same connection through which data is transmitted. In fact, in some embodiments, data received from the thermocycling unit (1) may lead to modulation of the electrical power transmitted from the smartphone. For instance, a temperature sensor (not shown) comprised by or attached to one of the thermal elements may submit data reporting a deviation of the respective thermal element's measured temperature from a desired value, upon which the power supplied to this element from the smartphone via the port (111) may be adjusted correspondingly.

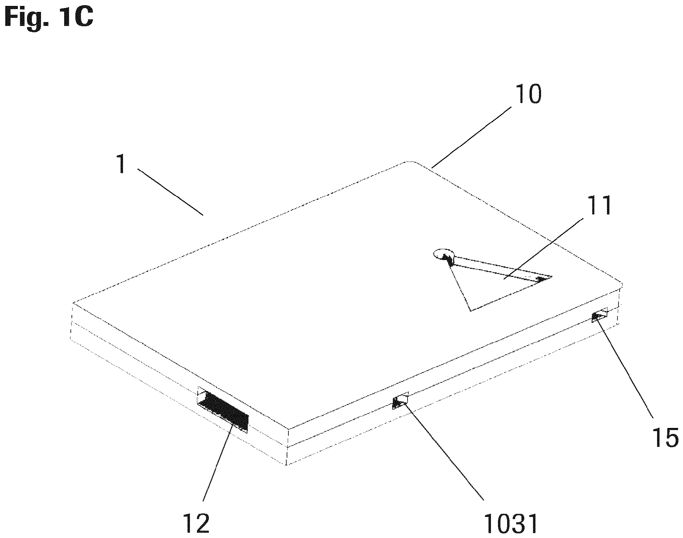

**Fig. 1C** shows the assembled housing (10) of the thermocycling unit (1) from the outside. The opening (1031) of the sample inlet port (103) is visible as well as the opening (12) of the USB port (111). Another opening (13) can be seen as a part of a docking mechanism for the corresponding smartphone (2). For instance, it may be a recess for a clip for clamping the thermocycling unit (1) to the smartphone (2). In other embodiments, the opening (13) may serve as a recess in which a lancet (not shown) is stored within the housing (10).

In addition to electrical coupling, the thermocycling unit (1) disclosed herein is also optically coupled to the smartphone (2) with which it interacts. In this context, an optical interface (11) is visible as part of the housing (10) in Fig. 1C. In this depiction, the interface (11) is configured to hold a light guiding element (121) such as a triangular prism. The latter is not shown in Fig. 1C for the sake of clarity.

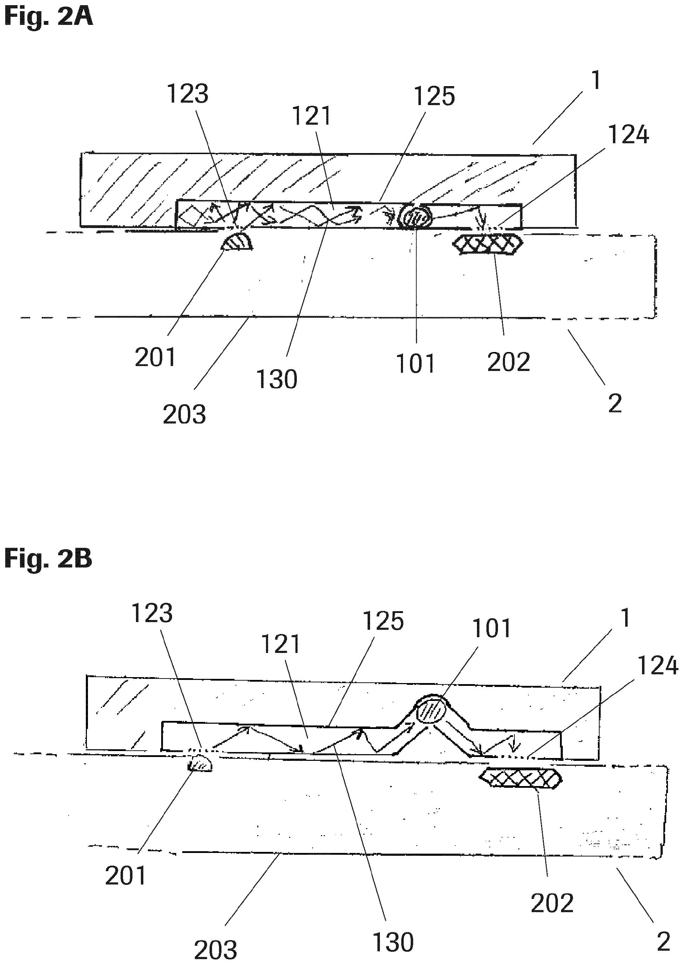

Exemplary embodiments of the optical coupling are depicted in **Figs. 2A** to **2D****.**

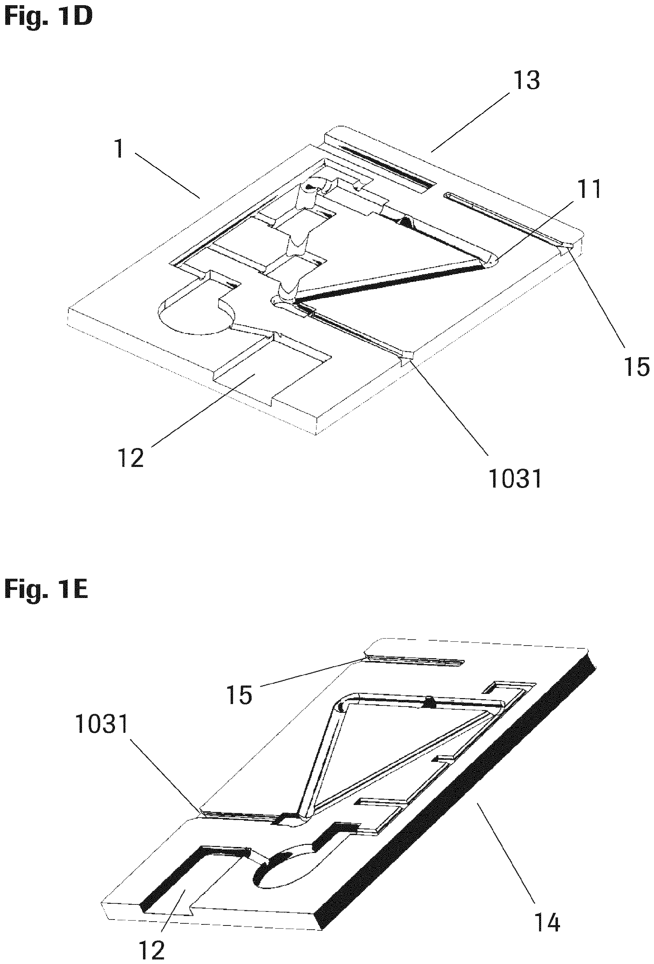

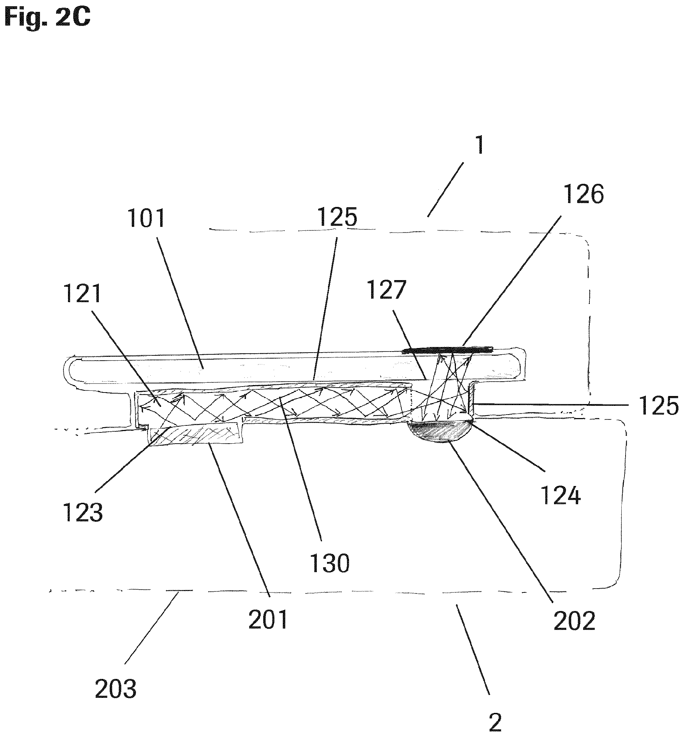

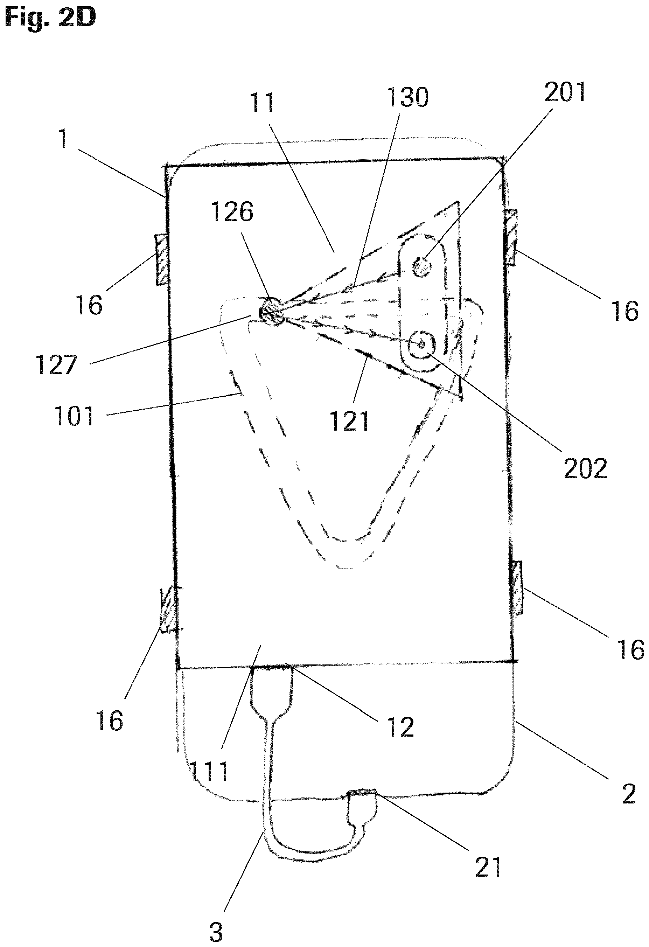

**Fig. 2A** represents a first exemplary embodiment and shows a cross-sectional side view of the optical path (120) constituted by the thermocycling unit (1) and the smartphone (2). In this highly schematic depiction, the thermocycling unit (1) is attached via its optical interface (11) to the face of the smartphone (2) containing the light source (24) and the camera (25). In the case of many contemporary smartphones, this is the backside of the smartphone, i.e., the face not containing the display. Advantageously, the light source (24) is usually located in proximity to the camera (25) in order to avoid loss of light intensity when taking a picture with the camera (202) and using the light source (201) as a flash. A light guiding element (121) of the optical path (120) can thus be dimensioned such that it will fit the majority of the commercially available smartphones, i.e., it will bridge the distance between the light source (24) and the camera (25). In some embodiments, the light guiding element (121) is a prism, such as a triangular prism as depicted in Fig. 1. Such a prism (121) confers the additional advantages of collecting light, such as scattered light, from the light source (201). Such light might otherwise be lost for the purpose of illuminating the reaction mixture. The prism (121) in this embodiment can be better appreciated in Fig.1 from the perspective view provided therein, since the cross section of the "lying" triangular prism (121) in Fig. 2 does not show the details of its geometry. The base of the triangle facing the light source (24) provides a relatively wide area in which scattered or otherwise undirected light can be reflected from the inner surface of the tapered sides of the triangle - in the direction of the light beam - and thereby be guided towards the tip of the triangle in whose vicinity the camera (25) is located. The sides, or the entire inner surface of the prism (121) may comprise a reflective coating (125) or the like, contributing to reducing the loss of light intensity.

The path of a light beam emitted by the light source (24) in Fig. 2A is represented by a series of arrows (130). In some embodiments, the light source (24) is a light-emitting diode (LED), an OLED, a halogen such as a xenon lamp, or the like. Typically, such light sources create white light, often composed of multiple monochromatic beams. In order to analyze the newly created nucleic acid in the reaction mixture, oftentimes light of a specific wavelength is exploited. An optical filter (123) located at the interface between the light source (24) and the prism (121) may be used for filtering the desired wavelength or wavelength range. Also, such a filter (123) may contribute to reducing undesired optical effects, for instance, by filtering out scattered ambient light. The reflective coating (125) contributes to guiding the light from the light source (24) on its intended path (130) towards and through the cyclic fluidic path (101). Depending on the detection method, the light beam may, for example, lead to excitation of a fluorescent dye associated with the nucleic acid to be analyzed. In such embodiments, the camera (25) detects light at the emission wavelength of the respective fluorophore, for instance, through a further optical filter (124) selectively permitting the transmission of the emission wavelength.

Turning to **Fig. 2B****,** another embodiment regarding the optical detection system is depicted. In essence, the majority of the elements are identical with the ones illustrated in Fig. 2A. It can, however, be gathered that the cyclic fluidic path (121) is slightly spatially shifted towards the upper outer rim of the thermocycling unit (1). Separating the cyclic fluidic path (121) in the manner of the depicted embodiments leads to more efficient guiding of the light beam to and through the reaction mixture.

**Fig. 2C** is a depiction of a third exemplary embodiment, viewed from the same perspective as in Figs. 2A and 2B. In this embodiment, the primary beam emitted from the light source (201) also passes through a first optical filter (123) into the body of the prism (121) and is reflected off the reflective coating (125) on the inner surface of the prism (121). Upon reaching the end of the prism (121) facing the camera (202), a part of the light beam passes through the cyclic fluidic path (101) via an optical window (127) by which the prism (121) is optically connected to the cyclic fluidic path (101) of the reaction channel (100). The light beam is then reflected by a mirror (126) located behind the distal inner wall of the cyclic fluidic path (101). The light having traversed the reaction mixture within the cyclic fluidic path (101) then reaches the camera (202) through the second optical filter (124).

**Fig. 2D** shows the embodiment of Fig. 2C from a perspective facing the backside of the smartphone (2) with the thermocycling unit (1) attached to it via clips (16). A USB adapter cable (3) establishes the transfer of both data and electrical power between the port (21) on the smartphone (2) and the USB port (111) including an opening (12) in the housing (10) of the thermocycling unit (1). The thermocycling unit (1) is depicted in a transparent manner for the sake of visibility of the underlying elements. The optical interface (11) allows for the light source (201) to transmit its light into and through the prism (121) such that it reaches the cyclic fluidic path (101) through the optical window (127) before being reflected by the mirror (126) behind the capillary (101). This part of the emitted light, represented by the arrows (130), then ultimately reaches the camera (202) of the smartphone.

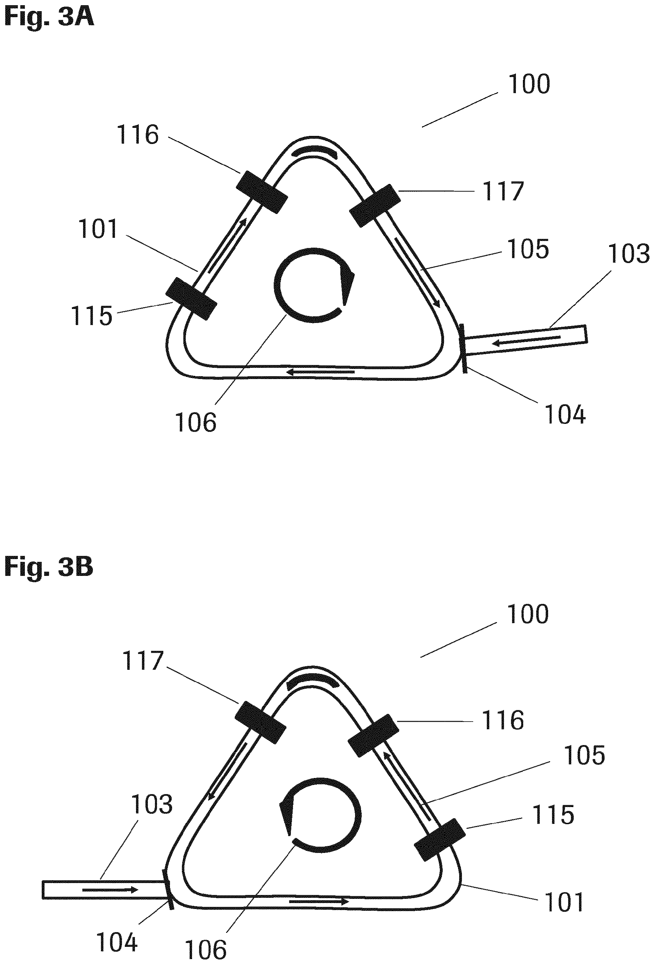

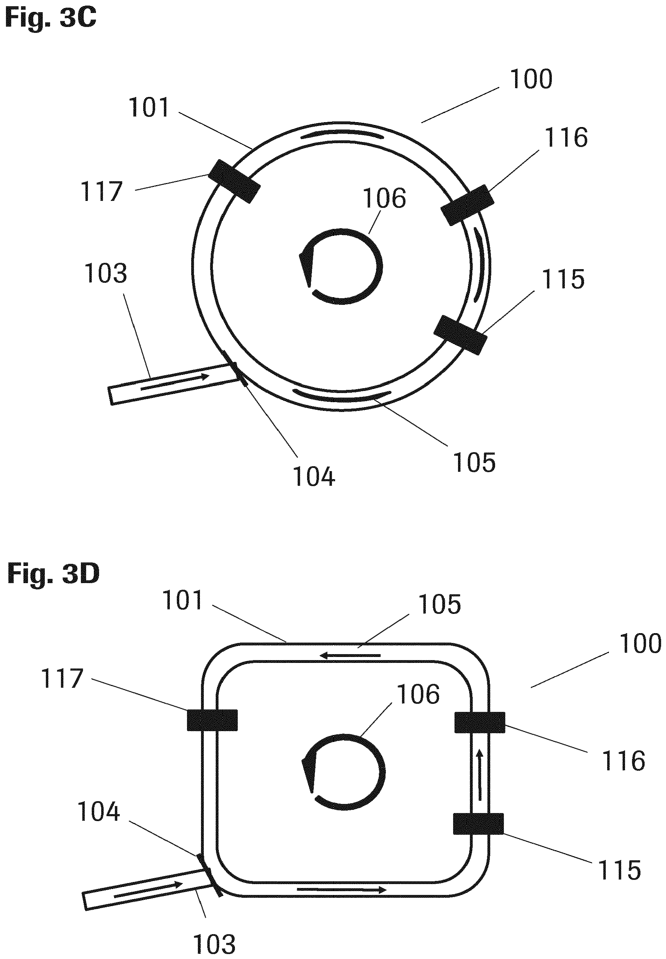

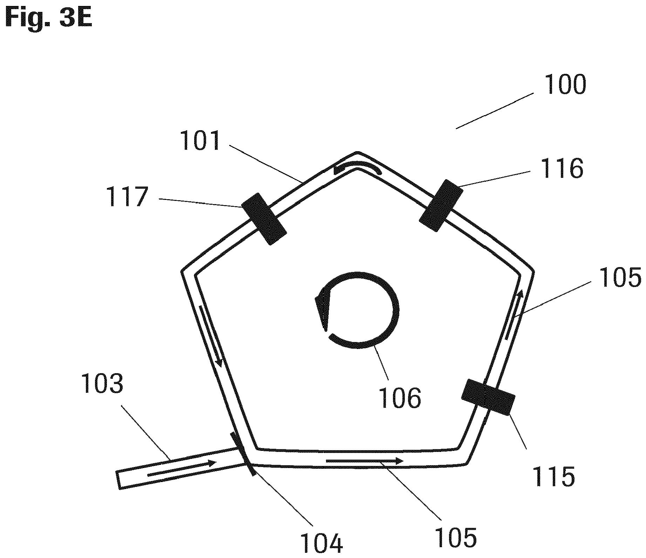

**Fig. 3** shows different embodiments of the reaction channel (100). These depictions exemplify the generation of the temperature gradient and the resulting fluidic motions.

In **Fig. 3A****,** the reaction channel (100) is configured so that the liquid sample is introduced via the sample inlet port (103) into a triangular cyclic fluidic path (101). The first thermal element (115), set at a temperature above 90 °C, serves for creating a denaturing zone. The second thermal element (116) is set at an annealing temperature of about 60 °C, while the third thermal element (117) is set at an elongation temperature of about 70 °C. Hence, the temperature difference between the first (115) and the second (116) thermal elements is greater than the difference between the second (116) and the third (117) or the first (115) and the third (117) thermal elements, respectively. Once the temperatures of the thermal elements are set, the liquid flow will follow the steepest temperature gradient. In the depicted embodiment, this will consequently be in a clockwise direction as indicated by the circular arrow (106) as well as the straight arrows (105). Further visible in Fig. 3A is a backflow preventer (104) contributing to keeping the reaction mixture inside the cyclic fluidic path (101). Such backflow preventer (104) may be embodied by, for example, a membrane, a frangible seal, a rubber re-closing itself after being pierced by a lancet, a valve, or the like. The skilled person is aware of suitable further means for preventing backflow.

**Fig. 3B** essentially depicts the embodiment of Fig. 3A viewed from the backside, or, else an embodiment with a mirror-inverted setup in which the liquid flow is counter-clockwise.

**Fig. 3C** shows a circular cyclic fluidic path (101) and **Fig. 3D** an essentially rectangular one. Turning to **Fig. 3E****,** it can be seen that also pentagonal shapes are conceivable. The person skilled in the art is capable of implementing further geometrical structures, depending on the available space or other circumstances.

It can be gathered from the depictions in Fig. 3 that it is advantageous to arrange the sample inlet port (103) in a manner that the injection of the sample is aligned with the flow direction following the thermal gradient. While a reverted injection is also possible, an aligned injection confers the benefits of not causing turbulences in the liquid flow and contributing to a smooth and steady movement of the reaction mixture along the cyclic fluidic path.

### Example

In the following, an experiment is described in order to illustrate a way to work the method described herein with a device and an analytical system described herein.

### Experimental Setup:

### Materials

### Thermocycling unit (corpus):

Thermocycling units according to two different embodiments described herein were built by 3D-printing, one set being a fully integrated thermocycling unit with a closed chassis, the other set having a re-openable housing for separate, insertable reaction channels.

### Reaction channel:

For the reaction channel, capillaries made of glass or polypropylene were locally heated and bent into the triangular shape used in this experiment. Loose ends were cut and the resulting cyclic fluidic path was rinsed thoroughly with water and an aqueous 70 % (V/V) ethanol solution. Further, the inside walls of the capillaries were hydrophobized using dichloro-dimethylsilane, thus rendering them chemically inert and preventing, for example, unspecific attachment of nucleic acids. The designated sample injection port was sealed with silicone, and a plasma separation membrane was placed at the interface of injection port and cyclic fluidic path. The silicone seal was pierced with a heated needle such as to create an inlet opening for a lancet for sample injection. As the inlet opening, due to the elastic properties of the employed silicone, was self-resealing, it also served as a backflow preventer.

### Electronic parts:

As a control unit, a commercially available Arduino UNO R3 chip assembly was used along with the Arduino kit Max6675 for controlling the thermal elements. The distinct temperatures were set as follows:

| | |
|---|---|
| First thermal element (denaturing): | 95 °C |
| Second thermal element (annealing): | 55 °C |
| Third thermal element (elongation): | 72 °C |

### Sample material:

For the sake of testing the PCR procedure, whole blood as well as urine samples were used, representing potential clinical or forensic samples, or the like.

Further, nucleic acids from the commercially available Crime Scene Investigator PCR Basics Kit (Bio-Rad) were employed. The PCR reagents provided in this kit were pre-filled into the reaction channel prior to the addition of the liquid sample.

### Sample preparation:

For isolating nucleic acids from some of the liquid samples, the commercially available kit "HP-System Viral Nucleic Acid Kit" (Roche Diagnostics, ID 3502295001) was used according to the manufacturer's instructions, along with the corresponding filter tubes (ID 3502295001). A part of the whole blood samples was prepared using the cobas® Plasma Separation Card (Roche Diagnostics, ID 8253480700).

### Detection:

The Crime Scene Investigator PCR Basics Kit included ethidium bromide as an intercalating dye. In some cases, detection was effected by withdrawing an aliquot from the reaction channel and separating it by means of conventional agarose gel electrophoresis followed by UV light-induced fluorescence detection. It is advantageous to use an optical path as disclosed herein, thereby exploiting the light source (flash) and the camera of the smartphone in use and creating an integrated system as disclosed herein. In some cases, pictures were taken continuously throughout the PCR, and based on the pixel density, the relative amount of nucleic acids was estimated. In some cases, the commercially available Android app "Vernier Spectral Analysis" was used for this purpose, but other suitable apps are available and known to the person of ordinary skill in the art.

### Smartphone:

The following two commercially available smartphones were used in connection with the thermocycling unit described herein, according to the instructions of the respective manufacturer:
- Samsung Galaxy S6 (released April 2015)
- Lenovo ZUK Z2 (released June 2016).

### Methods

The samples were provided depending on their origin. Urine was collected in a suitable vessel, whole blood was withdrawn using a lancet provided with the thermocycling unit described herein, and the nucleic acid samples from the commercial kit were provided in designated tubes.

The respective liquid sample was subjected to a sample preparation process as described above or introduced directly into the thermocycling unit attached to one of the smartphones mentioned above via the sample inlet port. Whole blood samples were filtered *in situ* via a plasma separation filter within the separation unit of the reaction channel, or in some cases externally before introduction into the sample inlet port.

The thermal elements were heated as indicated above in order to create a thermal gradient and the resulting liquid flow. The sample liquid introduced into the reaction channel was allowed to mix with the pre-disposed PCR reagent mix and to flow along the thermal gradient through the designated different temperature zones, thus effecting a polymerase chain reaction within the reaction channel. Detection of the amplified nucleic acids was performed as described above.

### Results

The principle of inducing a stable liquid flow within the reaction channel was shown by injection of an ink-based chemical dye. The liquid movement upon heating of the thermal elements of the embodiment of this example was visible via tracking of the dye.

Amplified nucleic acids were detectable in the samples designated as positive, either by gel electrophoresis or by pixel density measurements as described above, demonstrating the suitability of the method, device and system described herein for the amplification of nucleic acids.

## Claims

1. Thermocycling unit (1) for carrying out a Polymerase Chain Reaction (PCR) in connection with a smartphone (2) having a CPU, a power source, a display (203), a light source (201) and a camera (202), the thermocycling unit (1) comprising:
- a reaction channel (100) providing a cyclic fluidic path connected to a liquid sample inlet port (103) via a gate (102);
- a housing (10) comprising the liquid sample inlet port (103) and an optical interface (11) configured to be placed onto the light source (201) and the camera (202) of the smartphone (2), the optical interface (11) being connected to an optical path (120) within the thermocycling unit (1) for guiding light from the light source (201) via the reaction channel (100) to the camera (202) of the smartphone (2);
- a data transfer port (111a) and an energy transfer port (111b), wherein the data transfer port (111a) and the energy transfer port (111b) may be combined or separate;
- an electric circuitry (110) comprising a first (115) and a second thermal element (116) in thermal contact with the reaction channel (100), wherein the first thermal element (115) is spatially separated from the second thermal element (116), and wherein the first (115) and the second (116) thermal elements are configured to be set to different pre-determined temperature values such as to establish distinct temperature zones and a temperature gradient along the reaction channel (100);
- a docking mechanism for reversibly docking the thermocycling unit (1) to the smartphone (2).

2. The thermocycling unit (1) of claim 1, wherein the data transfer port (111a) and the energy transfer port (111b) are combined in a USB port (111).

3. The thermocycling unit (1) of any of the preceding claims, wherein the reaction channel (100) is pre-filled with PCR reagents.

4. The thermocycling unit (1) of any of the preceding claims, wherein the electric circuitry (110) further comprises a third thermal element (117) in thermal contact with the reaction channel (100), wherein the third thermal element (117) is spatially separated from the first (115) and the second (116) thermal elements.

5. The thermocycling unit (1) of claim 4, wherein the first thermal element (115) is configured to be heated to a PCR denaturing temperature (about 90 to 95 °C), the second thermal element (116) is configured to be heated to a PCR annealing temperature (about 50 to 65 °C), and the third thermal element (117) is configured to be heated to a PCR extending temperature (about 70 to 80 °C).

6. The thermocycling unit (1) of any of the preceding claims, wherein the gate (102) is a separation unit for a biological sample.

7. The thermocycling unit (1) of claim 5, wherein the separation unit (102) comprises a blood plasma separation filter (1022).

8. The thermocycling unit (1) of any of the preceding claims, wherein the first (115) and/or the second thermal element (116) are selected from the group consisting of a Peltier element, an electrodeposited thermoelectric element, and an electrical resistance element.

9. The thermocycling unit (1) of any of the preceding claims, wherein the docking mechanism for reversibly docking the thermocycling unit (1) to the smartphone (2) comprises one or more elements selected from the group consisting of a snap fit, a press fit, latches, and hooks.

10. The thermocycling unit (1) of any of the preceding claims, wherein the optical path (120) comprises one or more elements selected from the group consisting of a prism, a lens, a guiding rod, an optical filter, a diffraction grating, and a mirror.

11. Method for conducting a PCR on a liquid biological sample, the method comprising the steps of:
a) introducing the liquid biological sample into the liquid sample inlet port (103) of the thermocycling unit (1) of any of the preceding claims and docking the latter to a smartphone (2) having a CPU, a power source, a display (203), a light source (201) and a camera (202);
b) setting the temperatures of the first (115) and the second thermal element (116) of the thermocycling unit, by using the CPU and the power source of the smartphone (2), to different values and thereby establishing distinct temperature zones and a temperature gradient along the reaction channel (100);
c) cycling the liquid biological sample through the distinct temperature zones of the reaction channel (100) by using the temperature gradient for thermal convection, thereby conducting the PCR steps of denaturing, annealing and elongating within the distinct temperature zones;
d) detecting a nucleic acid amplification product of the PCR by guiding light emitted from the light source (201) of the smartphone (2) through the optical path (120) of the thermocycling unit (1) to the camera (202) of the smartphone (2) either during or after step c).

12. The method of claim 11, further comprising after step d) the step of processing the detection data and outputting a result to the display (203) of the smartphone (2).

13. The method of any of the claims 11 or 12, further comprising the step of pre-processing the liquid biological sample after insertion into the thermocycling unit (1) through the liquid sample inlet port (103), but before cycling the liquid biological sample through the reaction channel (100).

14. Analytical system for conducting a PCR, the system comprising:
- a thermocycling unit (1) of any of the claims 1 to 10;
- a smartphone (2) having a CPU, a power source, a display (203), a light source (201) and a camera (202).

15. Use of the thermocycling unit (1) of any of the claims 1 to 10 for conducting qualitative or quantitative realtime PCR.
